# EUROPEAN PATENT SPECIFICATION

(11) **EP 2 710 982 B1**
(45) Date of publication and mention of the grant of the patent: **31.08.2016**
(21) Application number: 12185404.6
(22) Date of filing: 21.09.2012
(51) Int. Cl.: A61F 2/36, A61F 2/40, A61F 2/30

(54) **Joint implant**
Gelenkimplantat
Implant d'articulation

(43) Date of publication of application: 26.03.2014
(73) Proprietor: WALDEMAR LINK GmbH & Co. KG, 22339 Hamburg (DE)
(72) Inventor: Link, Helmut D., 22397 Hamburg (DE)
(74) Representative: Hoffmann Eitle

(56) References cited:
- DE-A1- 4 304 022
- FR-A1- 2 715 568
- FR-A1- 2 810 232
- US-A1- 2007 118 229

## Description

### FIELD OF THE INVENTION

This invention relates to a joint implant for implantation into a bone. The implant comprises a stem having a distal end and a proximal end and at least two grooves forming a ridge between said grooves, wherein the grooves substantially extend along the longitudinal direction of the stem.

### BACKGROUND OF THE INVENTION

In general, there are two techniques for anchoring a joint implant in bone tissue. The first technique was developed by Charnley and is based on bone cement (PMMA) as a means for fixation of the implant within a bone cavity. However, the bone cement may be exposed to fatigue due to cyclic loads transferred through the implant whenever the patient is moving.

The second technique is to insert an implant with an osteoconductive surface into a bone cavity. Such an osteoconductive surface promotes ingrowth of the surrounding bone tissue. This eventually results in the implant being firmly attached to the bone tissue. It has been shown that implants fixed within the bone without using bone cement are less prone to loosening.

The process of bone ingrowth inter alia depends on the contact conditions between the bone tissue and the implant's surface. The contact between the bone tissue and the implant is established by preparing a bone cavity that generally corresponds to the shape of the implant. However, the implant is normally inserted into the bone cavity using an interference fit. Thus, sufficient bone density is needed to withstand the forces created by the interference fit and the daily loads applied by the patient. Moreover, the patient will have to move within a day or two after surgery to prevent adverse effects on muscles and tendons, which may result in a decreased stability of the joint replacement.

Bone ingrowth takes considerably more time than fixation of the implant by using PMMA. In addition, primary stability directly after surgery is critical in order to prevent micromotion having an adverse effect on bone ingrowth. However, if primary stability can be provided, bone tissue will form a durable interface with the implant. Gradually, a shift occurs from anchoring by primary stability generated by the above-mentioned interference fit or press-fit to anchoring by secondary stability achieved by firm attachment of the bone tissue to the implant's surface due to bone ingrowth.

In addition, the contact conditions can also be influenced by adapting the structural design of the implant, for example by altering its dimensions or surface structure to increase the contact surface to the cortical and/or cancellous bone tissue. This increase in contact area will result in a decreased contact pressure acting on the bone, avoiding the occurrence of bone resorption or fracture due to excessive loading.

Further, applying a tapered or wedge-shaped design to the implant in the direction of insertion will cause a press-fit of said implant within the bone tissue. The objective of the press-fit is to make use of the elasticity of the bone structure to create an interlock with the implant. Additional features such as ribs and grooves may also be used to fix the implant in the bone.

For example, US 2009/0299584 A1 discloses an implant that is provided with ridges that are intended to fix the implant in position. Further, the stem of the implant has a
wedge shape that provides initial stability when press-fitted into the bone cavity. US 2009/0299584 A1 also discloses a coating made of titanium beads in order to promote bone ingrowth. EP 0 169 976 is designed to improve bone ingrowth by proving a network of longitudinal ridges that are interrupted by transverse grooves. FR 2 715 568 A1 forms the basis of the two-part form of independent claim 1 and discloses the use of nacre in powder form mixed with blood during surgery. Said mixture fills up the grooves of an implant for promoting bone ingrowth.

In general, bone ingrowth may be improved by providing osteoconductive and/or osteoinductive surfaces by mechanical and chemical surface treatments or by the application of a coating. Often, these surface modifications at least partly promote bone ingrowth by providing an increased surface roughness.

In summary, anchoring an implant without bone cement generally involves two phases that complement each other. In the first phase, a press-fit is applied for primary stability to initiate bone ingrowth and to create a strong interface between the bone tissue and the implant, which in turn provides secondary long-term stability.

It is advantageous for the application of the press-fit if the implant rather slides along the cortical and/or cancellous bone tissue during insertion. Once fitted into the cavity, further compression is applied to deform the surrounding bone tissue elastically. For the creation of the press-fit it is advantageous that the implant has a smooth surface to avoid an abrasive effect of the implant's surface on the bone tissue during implantation. The abrasive effect may result in a decreased press-fit by damaging bone tissue and releasing elastic compressive tension of the bone tissue. In order to counteract this abrasive effect, a higher insertion force may be needed. This increase of force may locally result in higher compressive forces and increased bone resorption, which in turn increases the risk of loosening of the implant.

Despite having advantageous effects for secondary stability, an increased surface roughness turns out to be disadvantageous during the creation of a press-fit that is needed to promote bone ingrowth. Consequently, achieving both primary short-term stability and secondary long-term stability by modifying the surface of the implant represent contradictive objectives that have to be solved.

### SUMMARY OF THE INVENTION

The present invention addresses this objective problem and provides an implant that is able to create sufficient primary stability and at the same time has surface properties that promote bone ingrowth for long-term secondary stability. The implant according to the invention is defined in independent claim 1 with further aspects and preferred embodiments defined in the dependent claims.

Particularly, the present invention provides a joint implant for implantation into a bone. The joint implant comprises a stem having a proximal section and a distal section, at least two grooves substantially extending along the longitudinal direction of the stem, a first coating at least partially covering the surface of said elongated grooves, and a ridge, the ridge being formed in between adjacent grooves and having an apex surface. The apex surface of the ridge has an average surface roughness that is lower than the surface roughness of the first coating.

The surface roughness of the apex surface being lower than the external surface roughness of the first coating has the effect that the objective of primary and secondary stability is primarily achieved at specific predetermined locations of the implant. More specifically, the lower surface roughness of the ridge's apex surface primarily causes elastic compression of the bone tissue during insertion of the implant. Without wishing to be bound by theory, it is believed that the release of tension that enables the press-fit interlock between the implant and the bone due to damage of the bone tissue is avoided by the comparatively smooth apex surface.

The apex surface is the surface of the ridge facing away from the implant. If the groove forms an edge with said ridge, the apex surface is limited on its longitudinal sides by edges of the adjacent grooves.

It is to be understood that the press-fit may not only be achieved by the apex surface of the ridge but by the entire external surface of at least the proximal section without the surface of the grooves. This surface forming an envelope around the implant under the assumption that the implant had no grooves will in the following be referred to as envelope surface. In other words, the envelope surface describes a continuous surface that would be present without any elongated grooves. When referring to the envelope surface in terms of surface roughness, it is to be understood that the surface of the grooves and/or the first coating is not comprised.

The implant has at least two grooves with one ridge in between. It is to be understood that the implant may also have three grooves with two ridges in between and more preferably four grooves with three ridges in between. Due to the application of the first coating, the depth to width ratio of the grooves and/or manufacturing costs, it is advantageous if the number of grooves does not exceed twelve. Further, the aforementioned numbers of grooves are preferably situated on the same side of the implant.

The first coating situated on the surface of the elongated grooves is configured to promote ingrowth of bone tissue by having a higher average surface roughness in comparison to the apex surface. Preferably, only one layer of the first coating is applied. However, it is possible to apply more than one layer of the first coating, such as 2, 3, 4 or 5 layers.

The first coating is osteoconductive, i. e. it is used by osteoblasts as a framework upon which to spread and generate new bone. Although the surface roughness of the first coating increases the probability that local failure of the contacting bone tissue is caused, primary stability is still maintained by the interference fit of the apex surfaces with the bone tissue.

After implantation and as part of the healing process, the bone tissue will start to attach itself and to grow into the topology provided by the osteoconductive first coating. As result, an increasing secondary stability supplements the anchorage achieved by the press-fit and eventually provides a firm attachment of the implant to the bone.

The cavity within the bone tissue may be prepared with a tool such as a rasp or a milling device. Preferably, the cavity corresponds to the shape of the implant. However, the tool may be designed so that the bone cavity is slightly undersized causing an interference fit and may also be partially oversized causing comparatively low or even no contact with the bone tissue. In other words, the dimensions of the tool forming the bone cavity may be adapted to cause less interference with the surface of the first coating, wherein the remaining surface of at least said proximal section is objected to more interference. Thus, less contact pressure exists between the bone and the implant's surface at the first coating than at the remaining surface including the ridge's apex surface.

In relation to the first coating it may even be advantageous to select dimensions for the tool so that a bone cavity is formed that is configured to have no interference with the first coating.

Anatomically, the proximal section of the stem is being situated in a portion of the bone that comprises cancellous bone tissue surrounded by a cortical shell. The distal section of the stem on the other hand is designed to be placed in the medullary canal of the long bone.

The proximal section of the implant is configured to transfer the major share of joint loads acting on the implant into the bone tissue facing the implant. Thus, forces are primarily introduced into the bone tissue near the opening of the bone cavity to prevent the occurrence of stress shielding. During stress shielding, remodeling of the bone may result in bone resorption at the proximal end on the level of the bone cavity's opening and bone formation on the level of the distal section due to the stimulus of loads transferred by the distal section. The result may be the failure of the implant's anchorage.

The distal section of the implant, i.e. the section situated in the direction of insertion, primarily guides the implant during insertion and is configured to avoid the transfer of forces into the bone tissue.

Preferably, the first coating is applied along the entire length of each groove. However, only a portion of a groove may be coated. For example, if said groove extends into the distal section of the stem, the first coating may extend from the proximal end of the groove up to the end of the proximal section. In other words, the first coating and the groove is only provided within the proximal section. Hence, the risk of stress shielding is reduced since the loads acting on the implant are mainly transferred by the external surface of the proximal section.

Likewise, not every groove has to be coated with the first coating to achieve the above-mentioned effect. Although secondary stability may also be provided by the surface not having the first coating applied thereto, the strength and stiffness of the interface between bone and implant will be higher in the area of said first coating so that the joint loads acting on the implant will primarily be transferred where the coating is present. Thus, increasing the area of the first coating on groove's surface has the advantage to enhance the secondary stability of the implant.

In another preferred embodiment of the invention, the depth of the grooves is at least 1 mm, preferably at least 2 mm, even more preferably at least 3 mm.

Choosing such a depth for the elongated groove avoids that any damage that may be caused to the bone tissue, i. e. to cortical or cancellous bone in the groove, does not affect the elastic compression of bone facing the apex surface. Otherwise, high compressive forces may lead to resorption of bone tissue, which in turn may cause a delay in bone ingrowth.

In another preferred embodiment of the invention, the ridge has a width such that the ratio of the width of the groove to the width of the ridge is between 0.5 and 5, preferably 1.5 and 4.

This range for the ratio between the widths of the groove and the ridge has been found to provide sufficient distribution of the loads acting on the bone facing the apex surface. Moreover, this range also provides a contact area for the bone facing the first coating that is able to trigger enough bone ingrowth for anchoring the implant permanently, i.e. for providing secondary stability.

In another preferred embodiment at least one of the grooves is designed to open up in its longitudinal direction towards at least one of the proximal end and the distal end.

The groove opening up towards the distal end allows the creation of a cavity in the bone tissue comprising a shape that also generally follows the outline of the grooves of said implant. As a result, bone tissue will be located in the grooves after insertion of the implant. Preferably all grooves are opening up towards the distal end.

Further, a groove opening up towards the proximal end facilitates the insertion of additional bone material or a bone graft material in order to increase bone density at the interface to the implant. The additional bone material has been found to support the healing process and the formation of bone tissue with a higher bone density. Further, a groove opening up towards the proximal end of the implant provides an interface for a tool to assist in removal of the implant in case a revision surgery needs to be performed.

A groove opening up to either or both sides also makes machining of said groove easier, thus reducing production costs.

In another preferred embodiment of the invention, the cross-section of at least one of the grooves is constant and/or increases in width towards the apex surface of the ridge.

This results in the sides of the groove being parallel or opening up towards the apex surface of the ridge or the envelope surface and causes a constant or increasing cross-section of the bone material situated within the groove. Thus, the load distribution during the transfer of joint loads, in particular shear forces, can be better accommodated by the bone tissue, further increasing secondary stability. In addition, the supply of nutrients to the bone tissue and the removal of metabolites is enhanced.

In another preferred embodiment, the apex surface of the ridge has an average surface roughness of at least 2.5 µm.

The average surface roughness of the apex surface enhances its osteoconductive properties. Thus, secondary stability is eventually not only provided by the first coating but also on the surface of the ridge's apex.

The apex surface may also have an average surface roughness smaller than 2.5 µm, such as at least 1 µm or at least 2 µm. Further, the average surface roughness of the apex surface is lower than the average surface roughness of the first coating. The average surface roughness of the apex may have a maximum surface roughness of 12 um, preferably 9 µm, and more preferably 7 µm. Moreover, the surface roughness Ra is preferably in a range from about 1 µm to about 9 µm, more preferably of from about 2.5 µm to about 7 µm. Preferably, the remaining surface of at least the proximal section has the same properties as said apex surface.

An average surface roughness of at least 2.5 µm has been found advantageous for the design of the implant since osteoconductivity is increased compared to a polished surface and at the same time, significant abrasion is avoided. In other words, the friction of the implant on the bone during insertion does not adversely affect the bone tissue, whereas long-term, this average surface roughness also allows the provision of an interface that supports secondary stability. Further, this average surface roughness has been found to provide a good basis for a firm attachment of the first coating in the groove and may thus at least also be used for the grooves prior application of the first coating to save manufacturing costs.

In another preferred embodiment of the present invention, the coating of the grooves is a titanium plasma spray coating, preferably having an average surface roughness of about 15 µm to about 30 µm, more preferably of about 20 µm to about 25 µm.

A titanium plasma spray coating is known for its osteoconductive properties, i. e. to promote bone ingrowth, and at the same time has a structural integrity that is not compromised by the shear forces occurring during insertion of the implant along cortical and/or cancellous bone tissue. Further, titanium plasma spray coating is a highly developed manufacturing technique that allows for a selective application of the first coating on the groove's surface.

In another embodiment of the present invention, the first coating (14) has a thickness of 200 µm to 500 µm and/or a porosity of 20 % to 40 %, the pores preferably having a size of from 75 µm to 200 µm.

These surface characteristics have been shown to be advantageous for the bone formation activity of osteoblasts. Even more preferred is a thickness of the coating of 250 µm to 350 um, most preferred 300 µm. In addition or alternatively, the porosity is more preferably 25 % to 35 %. The size of the pores should be large enough to allow osteoblasts to deposit bone by entering these pores during bone formation.

In another embodiment of the present invention, at least the surface of the proximal section has a second coating.

The second coating of this embodiment has the advantage that it may act as filler so that the resulting, surface of the portion of the stem the second coating is applied to is smoother than the original surface of the implant and/or the first coating. Preferably, the second coating is in addition to being osteoconductive also bioresorbable. In other words, the second coating promotes bone ingrowth and is being resorbed after implantation, i. e. it is replaced by native bone tissue. Due to the resorption, the osteoconductive surface of the first coating and/or the remaining surface of the implant with the second coating will be exposed further promoting attachment of the implant by bone ingrowth.

The second coating may be less durable than the first coating. Less durable in the present invention means that a portion of the first coating may be worn off during insertion and/or the coating may be soluble over time.

Preferably, the second coating is situated in the proximal section of the stem but may also cover at least a portion of the distal section. Thus, on one hand the second coating avoids abrasion of bone tissue during implantation for an enhanced primary stability and on the other hand positively affects the formation of secondary stability.

In another embodiment of the present invention, the implant comprises a surface portion situated between the first coating in said groove and the apex surface of the ridge, wherein said surface portion has the same surface roughness as said apex surface.

Since primary stability is achieved by a press-fit, the ridge partially enters the bone tissue during elastic compression of the tissue, in particular cancellous bone, so that a portion of the groove directly adjacent to the apex surface or surface of the ridge may also come into contact with bone tissue. In order to prevent the compressed tissue to be damaged by abrasion that may occur during contact with said first coating, the peripheral surface portion, which is situated between the first coating and the apex surface, has a lower surface roughness than the first coating. Preferably, the peripheral surface portion has the same surface roughness as said apex surface.

In another preferred embodiment of the present invention, the longitudinal shape of the ridge is designed to follow the trajectory of insertion of the implant.

On the one hand, this facilitates the insertion of the implant since the ridges help guiding the implant on the correct trajectory into the cavity. The same may apply to the grooves. On the other hand it further allows that a portion of the bone cavity selected for the press-fit does not come into contact with the first coating. In other words, during insertion it can be avoided that bone tissue passed by the first coating is subsequently passed by the surface having a decreased average surface roughness or vice versa.

In another preferred embodiment, the grooves are formed on opposite sides of said implant.

This embodiment provides the additional anchoring effect of the present invention achieved by the combination of grooves with the first coating and the apex surface with a lower surface roughness on opposite sides of the implant. It is generally preferred to place the grooves on a side of the implant that has a comparatively big contact surface with the bone tissue. It is further preferred to place two grooves on each of opposite sides of said implant. The placement of the grooves may be symmetrical and/or may be adapted according to the load transfer of the joints loads through the implant into the bone tissue.

In another preferred embodiment, the implant further comprises a neck section formed at the proximal end of the stem and preferably a collar which at least partially surrounds said neck.

The collar of the implant supports the positioning of the implant within the bone by limiting the insertion of the implant up to a predetermined depth. In other words, the collar acts as a stop that contacts the outer circumference of the bone cavity to mark the end of insertion. Thus, the collar is situated adjacent to the proximal section of the stem. This facilitates to achieve a predetermined press-fit for primary stability and avoids damage to the bone tissue by inserting the implant too far.

If at least one groove is present that does not open up to the proximal direction, the collar is preferably situated adjacent to the proximal end of the at least one groove. In the latter case both the proximal end of the at least one groove as well as the collar may serve as a stop.

Generally, the design of the joint implant according to the present invention is preferably used for anchoring at least partial replacement of diarthrodial joints, in particular for diarthrodial joints that are adjacent to at least one long bone with a medullary canal. For example, the features of the present invention may be applied to artificial hip joints, artificial knee joints, artificial ankle joints, artificial shoulder joints, artificial wrist joints, artificial finger joints and artificial elbow joints.

### Further,

### BRIEF DESCRIPTION OF THE FIGURES

The accompanying figures describe exemplary embodiments of the present invention. In these figures, same features or features having the same function are denoted with the same reference signs.
Figure 1 shows a side view of a first embodiment of a joint prosthesis according to the present invention; and
Figure 2 shows a side view of a second embodiment of a joint prosthesis according to the present invention.

### DETAILED DESCRIPTION OF PREFERRED EMBODIMENTS

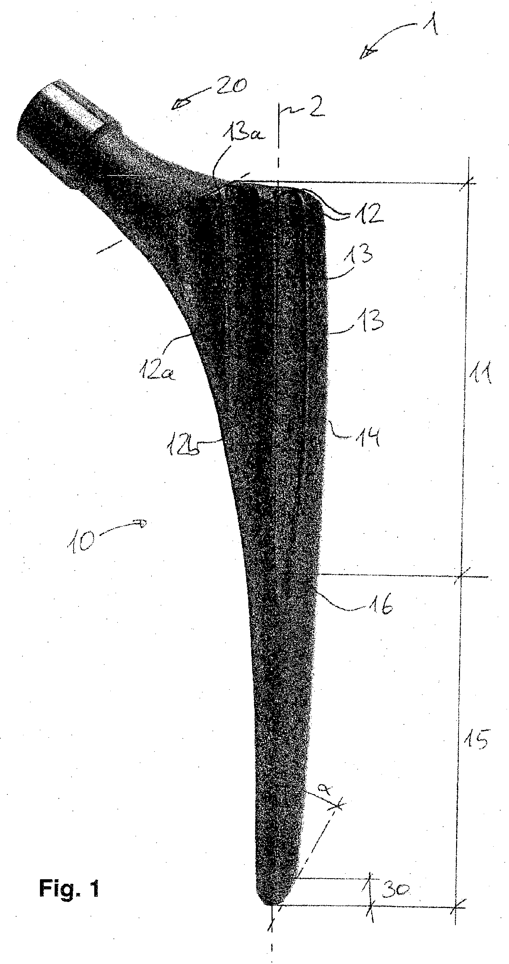

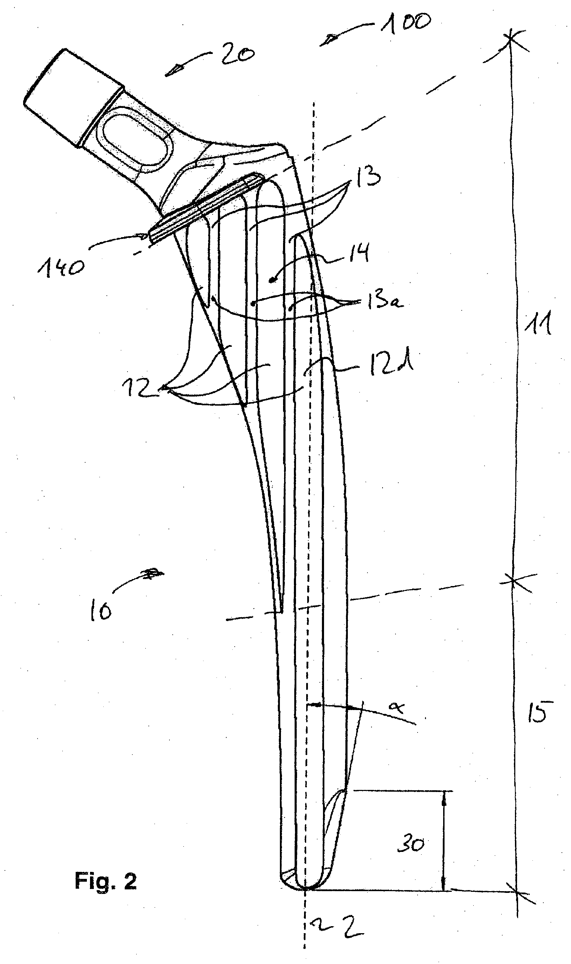

Fig. 1 shows a first preferred embodiment of an implant 1 according to the invention. More particularly, Fig. 1 shows a side view of a hip joint prosthesis 1 comprising features of the present invention to enhance both primary stability and secondary stability. Although the embodiment in Fig. 1 as well as the embodiment in Fig. 2 show exemplary embodiments of hip joint prostheses, the skilled person will be aware that the principles and features of this invention may also be applied to other joint prostheses for implantation into a long bone, such as artificial knee joints, artificial ankle joints, artificial shoulder joints, artificial wrist joints, artificial finger joints, and artificial elbow joints.

The artificial hip joint 1 of Fig. 1 comprises on its proximal side a neck 20 and distally to said neck a stem 10. The stem 10 of implant 1 comprises a proximal section 11 and a distal section 15.

At least the proximal section 11 of implant 1 is designed for providing primary and secondary stability in order to anchor the implant in bone tissue (not shown). Accordingly, the proximal section 11 is located and configured to be in contact with cancellous and/or cortical bone tissue after being implanted.

The distal section 15, on the other hand, is preferably and primarily for guiding and orienting the implant 1 during insertion into a bone cavity that has been prepared in the long bone. More specifically, the distal section 15 is dimensioned to be located in the medullary canal of the long bone after implantation. Due to the placement of the distal section 15 and its surface characteristics, the distal section 15 is preferably not configured to provide secondary stability.

The distal section 15 may be provided with a re-directing surface 30 at its distal end that is inclined by an angle α in relation to the longitudinal axis 2 of the implant 1. The cross-section of the distal section 15, also if excluding the section with the re-directing surface 30, decreases in the distal direction. However, the decrease in cross-section of the distal-section 15 is smaller than the decrease in cross-section of the proximal section 11 from its proximal end to its distal end, at least when excluding the grooves 12.

The major portion of said grooves 12 is preferably located in the proximal section 11. The elongated grooves 12 extend in the direction of the longitudinal axis 2 of the implant 1, i.e. in a proximal-distal direction. Preferably, the grooves run substantially parallel to each other, i. e. except for the end portions, where the cross section of the grooves changes due to opening up in its longitudinal direction or terminating within the implant's material.

Between said grooves 12 ridges 13 are formed respectively. Preferably, the apex surface or surface 13a of a ridge 13 is flush with the envelope surface of the stem 10 of the implant 1. The envelope surface is the external surface of said implant 1 without taking the grooves 12 into account, i. e. the implant 1 is assumed to be without grooves 12.

In Fig. 1, the ratio of the groove 12 to the width of the apex surface 13a at the height of the envelope surface is about 2.5:1, except for the end-portions of the grooves 12. This falls into the range as stated for one of the preferred embodiments from above, which is preferably between 0.5 and 5 and more preferably between 1.5 and 4.

Groove 12a in Fig. 1 is a non-continuous groove that opens up towards the distal end but is closed towards the proximal end. The groove 12a may substitute or assist the function of the collar 14 as shown for the implant 100 in Fig. 2 and described in detail below. More specifically, when inserting the implant 1 into the bone cavity, the proximal end of groove 12a may act as a stop to limit the depth of insertion of implant 1 into the long bone.

As can be seen for groove 12b, a groove 12 according to the invention may also open up to either or both ends, i.e. distally and proximally. A groove 12 opening towards its distal end generally facilitates a preparation of a bone cavity that generally corresponds to the shape of the stem 10 of the implant 1. Thus, a groove 12 of the implant 1 will preferably face a correspondingly formed protrusion of the bone tissue. If a groove 12 opens up to the proximal direction such as groove 12b, this opening may be used to insert additional bone material such as autologous bone material or bone graft material in order to increase bone density at the interface to implant 1. Moreover, a proximal opening of a groove 12 may also help in removing implant 1 in case of revision surgery by offering a structural interface to which a tool may be coupled to.

As can be seen at the ends of grooves 12 shown in Fig. 1, the cross-section of grooves 12 may gradually decrease due to the general shape or envelope surface of the implant 1 (at the distal end) or by exiting on an adjacent side of the implant (here: proximal side of implant 1). The grooves 12 are present on at least one side of an implant 1, the side preferably having the largest contact surface with bone tissue. Thus, in the exemplary embodiment of an implant shown in Figs. 1 and 2, i.e. an artificial hip joint, there are two sides having large contact surfaces, i.e. the anterior and posterior side of the implant 1. In other words, grooves are preferably included in stem 10 of implant 1 shown in Fig. 1 on two sides of implant 1, i. e. the one pointing to the reader and the one pointing away from the reader.

Consequently, a ridge 13 with a surface or apex surface 13a formed between grooves 12 on opposite sides may also be referred to as a ridge 13. However, in such a case in which a ridge 13 spans over at least two sides of the implant, at least restrictions concerning the width ratio between a ridge 13 and a groove 12 do not apply. Nonetheless, these ridges 13 preferably have the same surface characteristics as the apex surface 13a of ridges 13 situated on the same side of an implant.

The grooves 12 of implant 1 preferably have a depth of at least 1 mm, more preferably at least 2 mm, or even more preferably at least 3 mm. On the surface of the groove, a first coating 14 is applied that provides an external surface to the implant 1 promoting bone ingrowth. The first coating 14 may entirely cover the surface of a groove 12 or, like in Fig. 1, cover a portion of such a groove. In this respect, it is preferred, that the first coating 14 covers at least 50%, more preferably at least 70%, most preferred at least 80% of a groove's surface, at least in the proximal section 11.

In a preferred embodiment and as shown in Fig. 1 a groove 12 may have a peripheral surface portion 16 running along the circumference of the groove 12 on which no first coating 14 is applied to. As already described above, this prevents bone tissue situated next to an apex surface 13a of a ridge 13 to be damaged upon insertion of the implant. Thus, the risk of an adverse effect on the press-fit, which is responsible for the primary stability, is at least reduced.

In general, a durable coating is to be chosen for the first coating 14 that is able to withstand the shear forces exerted upon the coating when inserting implant 1 into the bone cavity. In other words, the first coating is configured to not wear off during or after implantation into the long bone. It should also not dissolve during or after implantation. The first coating 14 is an osteoconductive coating. Preferably, the first coating 14 is a titanium plasma sprayed coating.

Further, the first coating 14 has a higher average surface roughness (Ra) than the apex surface 13a of a ridge 13. More specifically, the surface of the first coating 14 has a surface roughness of about 15 µm to about 30 um, preferably of about 20 µm to about 25 µm.

On the other hand, the apex surface 13a has a surface roughness Ra of about 1 um to 9 µm, preferably of about 2.5 µm to 7 µm. Preferably, the surface roughness of the apex, surface 13a also applies to the remaining surface of at least the proximal section 11 of implant 1, for example, to provide a surface that is configured for a firm attachment of the first coating when said coating is applied. In this respect, an average surface roughness of at least 2.5 µm is preferred as a basis for the first coating.

As already described above, the increased average surface roughness of the first coating 14 promotes bone ingrowth to achieve secondary stability. In contrast, the average surface roughness of the ridge's surface primarily facilitates insertion of the implant without causing damage to the bone tissue. Preferably, this also applies to the remaining envelope surface of at least the proximal section 11 of implant 1. Concerning the effect resulting from the interplay between the first coating 14 and the apex surface 13a of ridge 13 as well as the remaining envelope surface of at least a proximal section 11, it is referred to the description above.

Although not shown in Fig. 1, a second coating may be applied to the surface of the implant that additionally promotes bone ingrowth. The second coating will at least be applied to the proximal section 15 including the surface of the grooves and/or the first coating. Preferably, the second coating is applied to the entire surface of stem 10.

The second coating may have osteoinductive properties, i.e. bone ingrowth is additionally, or alternatively, stimulated by providing growth factors in order to trigger or support bone formation or bone ingrowth by chemical signals.

The material of implant 1 shown in Fig. 1 is a titanium alloy. Titanium is known for its high biocompatibility and its advantageous characteristics for bone ingrowth. However, other alloys may be used known from the prior art to produce an implant 1 according to the invention.

Fig. 2 shows a second embodiment of an implant according to the invention. Same features or features having the same function are denoted with the same reference signs. Thus, the detailed description of these features is not repeated. One structural difference of implant 100 shown in Fig. 2 is collar 140 and groove 12d that extends from the proximal section 11 throughout the distal section 15 to the distal tip of implant 100. Preferably, the portion of the groove 12d located in the distal section 15 has no first coating applied thereto.

Collar 140 acts as stop when inserting implant 100 into the bone cavity. If the bone cavity is formed to correspond to the shape of at least proximal section 11 of implant 100, the grooves 12 will at least partially receive bone tissue. As shown in Fig. 2, three of the elongated grooves extend proximally up to collar 140. Since they do not open up in the proximal direction, these grooves 12 also act as a stop besides the collar 140. Thus, on top of collar 140 and like in the previous embodiment of implant 1, the grooves 12 assist in achieving a predetermined press-fit of the implant 100 during implantation so that at least the surface pressure acting on bone tissue that faces the apex surface 13a of ridges 13 is prevented from passing a predetermined threshold causing adverse bone resorption and the risk of loosening.

Independent of any of the embodiments in the detailed description or the set of claims, the proximal section of the implant's stem is situated near the joint surface, whereas the distal section is situated away from the joint surface. It is also to be understood that preferably the shape of the implant is formed by grooves. However, it is also possible to form said grooves by attaching ribs to the surface of the joint implant.

## Claims

1. Joint implant (1; 100) for implantation into a bone, comprising:
a stem (10) having a proximal section (11) and a distal section (15);
at least two grooves (12) substantially extending along the longitudinal direction of the stem (10); and **characterized by**
a first coating (14) at least partially covering the surface (14) of said elongated grooves (12); and
a ridge (13), the ridge being formed in between adjacent grooves (12) and having an apex surface (13a);
wherein the apex surface (13a) has an average surface roughness that is lower than the average surface roughness of the first coating (14).

2. Implant (1; 100) according to claim 1,
wherein the grooves (12) have a depth of at least 1 mm, preferably at least 2 mm, even more preferably at least 3 mm.

3. Implant (1; 100) according to claim 1 or 2,
wherein the ridge has a width such that the ratio of the width of the groove (12) to the width of the ridge (13) is between 0.5 and 5, preferably between 1.5 and 4.

4. Implant (1; 100) according to any of the preceding claims, wherein at least one of the grooves (12) is designed to open up in its longitudinal direction towards at least one of the proximal end and the distal end.

5. Implant (1; 100) according to any of the preceding claims, wherein the cross-section of at least one of the grooves (12) is constant and/or increases in width towards the apex surface (13a) of the ridge (13).

6. Implant (1; 100) according to any of the preceding claims, wherein the apex surface (13a) of the ridge (13) has an average surface roughness of at least 2.5 µm.

7. Implant (1; 100) according to any of the preceding claims, wherein the first coating (14) of the grooves (12) is a titanium plasma spray coating, preferably having an average surface roughness of about 15 µm to about 30 µm, more preferably of about 20 µm to about 25 µm.

8. Implant (1; 100) according to any of the preceding claims, wherein the first coating (14) has a thickness of 200 µm to 500 µm and/or porosity of 20 % to 40 %, the pores preferably having a size of 75 µm to 200 µm.

9. Implant (1; 100) according to any of the preceding claims, wherein at least a portion of the surface of the proximal section (11) has a second coating.

10. Implant (1; 100) according to any of the preceding claims, further comprising a surface portion (16) situated between the first coating (14) in said groove (12) and the apex surface (13a) of the ridge (13), wherein the surface portion (16) preferably has the same surface roughness as said apex surface (13a).

11. Implant (1; 100) according to any of the preceding claims, wherein the longitudinal shape of the ridge (13) is designed to follow the trajectory of insertion of the implant (1; 100).

12. Implant (1; 100) according to any of the preceding claims, wherein the grooves (12) are formed on opposite sides of said implant (1; 100).

13. Implant (1; 100) according to any of the preceding claims, further comprising a neck section (20) formed at the proximal end of the stem (10) and preferably a collar (140), which at least partially surrounds said neck section (20).

## Patentansprüche

1. Gelenkimplantat (1; 100) zur Implantation in einen Knochen; umfassend:
einen Schaft (10) mit einem proximalen Abschnitt (11) und einem distalen Abschnitt (15);
wenigstens zwei Einkerbungen (12), die sich im Wesentlichen entlang der Längsrichtung des Schafts (10) erstrecken; und
**gekennzeichnet durch**
eine erste Beschichtung (14), die die Oberfläche (14) der langgestreckten Einkerbungen (12) wenigstens teilweise bedeckt; und
eine Rippe (13), wobei die Rippe zwischen benachbarten Einkerbungen (12) geformt ist und eine Scheitelfläche (13a) aufweist;
wobei die Scheitelfläche (13a) eine durchschnittliche Oberflächenrauigkeit aufweist, die geringer als die durchschnittliche Oberflächenrauigkeit der ersten Beschichtung (14) ist.

2. Implantat (1; 100) nach Anspruch 1,
wobei die Einkerbungen (12) eine Tiefe von mindestens 1 mm, vorzugsweise von mindestens 2 mm, besonders bevorzugt von wenigstens 3 mm aufweisen.

3. Implantat (1; 100) nach Anspruch 1 oder 2,
wobei die Rippe eine solche Breite aufweist, dass das Verhältnis der Breite der Einkerbung (12) zur Breite der Rippe (13) zwischen 0,5 und 5, vorzugsweise zwischen 1,5 und 4 beträgt.

4. Implantat (1; 100) nach einem der vorhergehenden Ansprüche, wobei wenigstens eine der Rippen (12) ausgelegt ist, sich in ihrer Längsrichtung zumindest zum proximalen Ende und/oder zum distalen Ende hin zu öffnen.

5. Implantat (1; 100) nach einem der vorhergehenden Ansprüche, wobei der Querschnitt von wenigstens einer der Einkerbungen (12) konstant ist und/oder in der Breite zur Scheitelfläche (13a) der Rippe (13) hin zunimmt.

6. Implantat (1; 100) nach einem der vorhergehenden Ansprüche, wobei die Scheitelfläche (13a) der Rippe (13) eine durchschnittliche Oberflächenrauigkeit von wenigstens 2,5 µm aufweist.

7. Implantat (1; 100) nach einem der vorhergehenden Ansprüche, wobei die erste Beschichtung (14) der Einkerbungen (12) eine Titan-Plasmasprühbeschichtung ist, die vorzugsweise eine durchschnittliche Oberflächenrauigkeit von ungefähr 15 µm bis ungefähr 30 µm, insbesondere von ungefähr 20 µm bis ungefähr 25 µm aufweist.

8. Implantat (1; 100) nach einem der vorhergehenden Ansprüche, wobei die erste Beschichtung (14) eine Dicke von 200 µm bis 500 µm und/oder eine Porosität von 20% bis 40% aufweist, wobei die Poren vorzugsweise eine Größe von 75 µm bis 200 µm aufweisen.

9. Implantat (1; 100) nach einem der vorhergehenden Ansprüche, wobei wenigstens ein Teil der Oberfläche des proximalen Abschnitts (11) eine zweite Beschichtung aufweist.

10. Implantat (1; 100) nach einem der vorhergehenden Ansprüche, ferner umfassend einen Oberflächenabschnitt (16), der zwischen der ersten Beschichtung (14) in der Einkerbung (12) und der Scheitelfläche (13a) der Rippe (13) angeordnet ist, wobei der Oberflächenabschnitt (16) vorzugsweise dieselbe Oberflächenrauigkeit wie die Scheitelfläche (13a) aufweist.

11. Implantat (1; 100) nach einem der vorhergehenden Ansprüche, wobei die Längsgestalt der Rippe (13) ausgelegt ist, der Bewegungsbahn der Einführung des Implantats (1; 100) zu folgen.

12. Implantat (1; 100) nach einem der vorhergehenden Ansprüche, wobei die Einkerbungen (12) auf gegenüberliegenden Seiten des Implantats (1; 100) geformt sind.

13. Implantat (1; 100) nach einem der vorhergehenden Ansprüche, ferner umfassend einen Halsabschnitt (20), der am proximalen Ende des Schafts (10) geformt ist, und vorzugsweise einen Kragen (140), der den Halsabschnitt (20) wenigstens teilweise umgibt.

## Revendications

1. Implant articulaire (1 ; 100) destiné à être implanté dans un os, comprenant :
une tige (10) comportant une section proximale (11) et une section distale (15) ;
au moins deux rainures (12) s'étendant essentiellement le long de la direction longitudinale de la tige (10) ; et
**caractérisé par**
un premier revêtement (14) recouvrant au moins partiellement la surface (14) desdites rainures allongées (12) ; et
une arête (13), l'arête étant formée entre des rainures (12) adjacentes et comportant une surface de sommet (13a) ;
la surface de sommet (13a) présentant une rugosité de surface moyenne qui est inférieure à la rugosité de surface moyenne du premier revêtement (14).

2. Implant (1 ; 100) selon la revendication 1, dans lequel les rainures (12) présentent une profondeur d'au moins 1 mm, de préférence d'au moins 2 mm, encore plus préférablement d'au moins 3 mm.

3. Implant (1 ; 100) selon la revendication 1 ou 2, dans lequel l'arête présente une largeur telle que le rapport de la largeur de la rainure (12) à la largeur de l'arête (13) est situé entre 0,5 et 5, de préférence entre 1,5 et 4.

4. Implant (1 ; 100) selon l'une quelconque des revendications précédentes, dans lequel au moins une des rainures (12) est conçue pour s'ouvrir dans sa direction longitudinale vers au moins l'une de l'extrémité proximale et de l'extrémité distale.

5. Implant (1 ; 100) selon l'une quelconque des revendications précédentes, dans lequel la section transversale d'au moins une des rainures (12) est constante et/ou augmente en largeur vers la surface de sommet (13a) de l'arête (13).

6. Implant (1 ; 100) selon l'une quelconque des revendications précédentes, dans lequel la surface de sommet (13a) de l'arête (13) présente une rugosité de surface moyenne d'au moins 2,5 µm.

7. Implant (1 ; 100) selon l'une quelconque des revendications précédentes, dans lequel le premier revêtement (14) des rainures (12) est un revêtement de titane déposé par projection au plasma, présentant de préférence une rugosité de surface moyenne d'environ 15 µm à environ 30 µm, plus préférablement d'environ 20 µm à environ 25 µm.

8. Implant (1 ; 100) selon l'une quelconque des revendications précédentes, dans lequel le premier revêtement (14) présente une épaisseur de 200 µm à 500 µm et/ou une porosité de 20 % à 40 %, les pores présentant de préférence une taille de 75 µm à 200 µm.

9. Implant (1 ; 100) selon l'une quelconque des revendications précédentes, dans lequel au moins une partie de la surface de la section proximale (11) comporte un second revêtement.

10. Implant (1 ; 100) selon l'une quelconque des revendications précédentes, comprenant en outre une partie de surface (16) située entre le premier revêtement (14) dans ladite rainure (12) et la surface de sommet (13a) de l'arête (13), dans lequel la partie de surface (16) présente de préférence la même rugosité de surface que ladite surface de sommet (13a).

11. Implant (1 ; 100) selon l'une quelconque des revendications précédentes, dans lequel la forme longitudinale de l'arête (13) est conçue pour suivre la trajectoire d'insertion de l'implant (1 ; 100).

12. Implant (1 ; 100) selon l'une quelconque des revendications précédentes, dans lequel les rainures (12) sont formées sur des côtés opposés dudit implant (1 ; 100).

13. Implant (1 ; 100) selon l'une quelconque des revendications précédentes, comprenant en outre une section de col (20) formée au niveau de l'extrémité proximale de la tige (10) et de préférence une collerette (140) qui entoure au moins partiellement ladite section de col (20).
